# FASCICULE DE BREVET EUROPEEN

(11) **EP 0 446 161 B1**
(45) Date de publication et mention de la délivrance du brevet: **06.09.1995**
(21) Numéro de dépôt: 91460008.5
(22) Date de dépôt: 07.03.1991
(51) Int. Cl.: A61B 5/11, A61N 1/18

(54) **Cuve à organe isolé, notamment pour recevoir la préparation neurobiologique d'un invertébré tel que la blatte, et ensemble d'étude comprenant une telle cuve**
Untersuchungseinheit mit einem Gefäss für ein neurobiologisches Präparat, insbesondere von einem Evertebrat, wie z.B. einer Schabe
Examination unit comprising a receptacle for a neurobiologic preparation, in particular from an invertebrate such as a cockroach

(30) Priorité: 07.03.1990 FR 9003075
(43) Date de publication de la demande: 11.09.1991
(73) Titulaire: UNIVERSITE DE RENNES I, F-35000 Rennes (FR)
(72) Inventeur: Callec, Jean-Jacques, F-35700 Rennes (FR); Hue, Bernard, F-49000 Angers (FR)
(74) Mandataire: Corlau, Vincent

(56) Documents cités:
- DE-A- 2 256 859
- FR-A- 1 209 081
- IEEE TRANSACTIONS ON BIO-MEDICAL ENGINEERING. vol. BME25, no. 5, septembre 1978, New York US; pages 479-481; D.E. Brodnick et al.: "Laminated Electrodes for Biopotential Studies"
- MEDICAL AND BIOLOGICAL ENGINEERING AND COMPUTING. vol. 17, no. 5, septembre 1979, STEVENAGE GB pages 583 - 592; J.S. PETROFSKY: "Constant-velocity contractions in skeletal muscle by sequential stimulation of muscle efferents"
- Proc. 7th Ann. Conf. of the IEEE/Engineering in Med. and Biol. Society 30 septembre 1985, Chicago,Ill.(USA) pages 69 - 73; C.M. COMER: "Processing Spatial Sensory Information in an Insect CNS"

## Description

Le domaine de l'invention est celui des dispositifs de réalisation d'expériences de biologie, essentiellement à des fins didactiques ou de recherche.

Plus précisément, l'invention concerne un dispositif d'étude de la neurophysiologie des invertébrés, au moyen d'une cuve électrophysiologique destinée à recevoir une préparation neurobiologique extraite d'un invertébré.

De façon connue, il existe des cuves à organes isolés adaptés aux nerfs sciatiques de vertébrés permettant d'étudier l'excitabilité du nerf et la conduction nerveuse par le biais de l'enregistrement des potentiels d'action déclenchés par stimulation électrique. Par exemple, le brevet français n° 2.617.702 au nom de Jeulin concerne un tel dispositif adapté au nerf sciatique de la grenouille. Le document FR-A-1 209 081 décrit un appareil pour réaliser simultanément l'enregistrement mécanique et électrique de réactions d'un organe de dimension variable dans une atmosphère controlée. Cependant, un décret de loi français (n° 87 - 848) interdit d'expérimenter sur des espèces animales vertébrées vivantes pour des raisons d'ordre éthique dans certaines conditions et notamment dans des établissements d'enseignement secondaire.

De plus, les dispositifs connus ne permettent pas d'étudier d'autres caractéristiques du système nerveux (activités sensorielles et transmission synaptique notamment).

La présente invention a notamment pour objectif de pallier ces inconvénients.

Un premier objectif de l'invention est de présenter un dispositif électrophysiologique simple adapté à l'analyse neurophysiologique chez un invertébré.

Un deuxième objectif de l'invention est de fournir un tel dispositif qui permette l'étude de l'excitabilité nerveuse, de la conduction nerveuse, de l'activité sensorielle (mécanoréception) et de la transmission synaptique neuro-neuronale de type chimique dans une telle préparation neurobiologique.

Un troisième objectif de l'invention est d'adapter un tel dispositif à un insecte du type de la blatte.

Ces objectifs, ainsi que d'autres qui apparaîtront par la suite, sont atteints selon l'invention grâce à une cuve à organe isolé pour préparation neurobiologique, du type comprenant une plaque horizontale d'extension d'une préparation neurobiologique, notamment destinée à un usage d'enseignement ou de recherche, coopérant avec des moyens d'alimentation de la préparation en liquide physiologique, et des moyens de stimulation électrique et/ou de lecture des signaux bioélectriques de la préparation, les moyens de stimulation et/ou de lecture comprenant au moins une électrode mixte, constituée d'une rainure creusée dans la plaque de réception de la préparation, sensiblement transversale à la direction d'extension de la préparation, la rainure communiquant avec un réservoir-tampon de liquide physiologique conducteur recevant un fil conducteur électrique de connexion aux moyens de stimulation et/ou de lecture.

Une électrode mixte est constituée par une rainure contenant du liquide physiologique et un fil conducteur.

Ladite électrode mixte a pour fonction de transmettre des signaux électriques entre la préparation à étudier et un (ou des) appareil(s) de lecture et/ou entre l'appareil de stimulation et la préparation. Ce moyen de transmission de signaux électriques présente notamment l'avantage d'être simple à mettre en oeuvre et de permettre un accès aisé à différents points de la préparation.

Préférentiellement, les moyens d'alimentation de la préparation en liquide physiologique sont constitués par au moins une rainure creusée dans la plaque de réception de la préparation, sensiblement transversale à la direction d'extension de la préparation, communicant avec un réservoir-tampon de stockage du liquide physiologique.

Avantageusement, les moyens d'alimentation de la préparation en liquide physiologique comprennent au moins une cuvette creusée dans la plaque de réception de la préparation, destinée chacune à recevoir une extrémité de la préparation, et contenant une réserve de liquide physiologique.

Selon un mode de mise en oeuvre préférentiel de l'invention, la cuve comporte une rainure creusée dans la plaque de réception de la préparation, sensiblement transversale à la direction d'extension de la préparation, et comportant une zone de réception de la synapse de la préparation, ladite rainure communiquant avec des moyens d'alimentation continue de la zone de réception de la synapse par un liquide physiologique à effet neurochimique en écoulement continu.

Préférentiellement, la cuve comporte une pluralité de rainures creusées dans la plaque de réception de la préparation, sensiblement parallèles entre elles et transversales à la direction d'extension de la préparation, les rainures comprenant d'une part une rainure centrale comprenant une zone de réception de la synapse de la préparation, et d'autre part au moins quatre rainures d'électrode de part et d'autre de la rainure centrale.

Avantageusement, les fils conducteurs électriques constitutifs des électrodes sont terminés chacun par une prise de connexion d'appareils, disposées de façon contigüe sur une plaque frontale commune de connexion.

Selon un mode de mise en oeuvre préférentiel de l'invention, les prises de connexion sont connectables sélectivement par paires, aux moyens de stimulation et/ou de lecture, selon un grand nombre de combinaisons d'appariement.

Avantageusement, la cuve comprend une potence support de suspension, à hauteur réglable, d'un réservoir de liquide formant les moyens d'alimentation en continu de la rainure comprenant une zone de réception de la synapse de la préparation.

De façon préférentielle, une telle cuve est appliquée à la réception de la préparation neurobiologique de la blatte, comprenant : "cerques, nerfs cercaux, synapse du sixième ganglion abdominal, chaîne nerveuse".

Deux espèces de blattes peuvent être notamment utilisées : Periplaneta americana et Blabera craniifer. Le choix de ces insectes est dicté par les avantages suivants:
- il s'agit d'invertébrés. Cela permet d'éviter l'écueil d'ordre éthique ;
- ils sont d'un élevage facile. Cela les rend facilement disponibles, en grand nombre et à un prix de revient négligeable ;
- ils fournissent une préparation neurobiologique facile à disséquer, à mettre en place, et à maintenir en survie dans la cuve selon la présente invention;
- ils permettent une analyse neurophysiologique étendue et de bonne qualité.

L'invention concerne également un ensemble complet, notamment à usage didactique ou scientifique, constitué d'une cuve telle que caractérisée précédemment, associée à des appareils de stimulation et/ou de lecture et d'exploitation électrique.

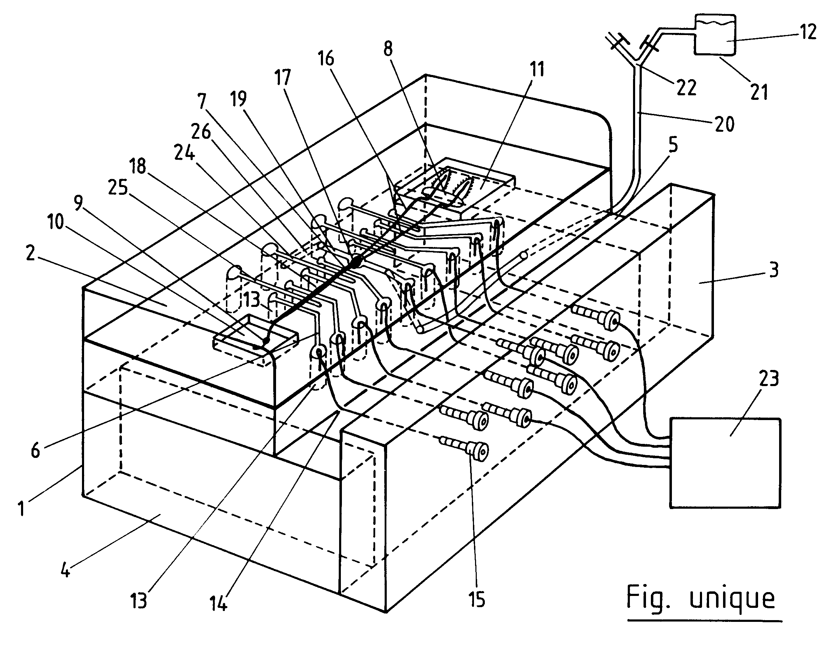

D'autres caractéristiques et avantages de la présente invention apparaîtront à la lecture de la description suivante d'un mode de mise en oeuvre préférentiel de l'invention, donné à titre illustratif et non limitatif et du dessin annexé représentant une cuve selon l'invention.

La figure annexée représente un mode de mise en oeuvre préférentiel de l'invention consistant en une cuve 1 en plexiglas constituée par une plaque horizontale 2, une plaque frontale 3 et deux plaques latérales 4,5.

La plaque horizontale 2 présente un certain nombre de rainures 6 sensiblement transversales à la direction d'extension de la préparation 7. Selon le mode de réalisation préférentiel choisi, la préparation 7 est une préparation neurobiologique d'une blatte adulte préalablement disséquée.

Les extrémités 8,9 de la préparation 7 reposent chacune dans une cuvette 10,11.

Chaque rainure 6 contient un liquide physiologique 12 conducteur et les extrémités de chaque rainure 6 sont chacune terminées par un réservoir 13 contenant le même liquide physiologique 12. Un des réservoirs de chaque rainure contient une extrémité d'un fil conducteur 14 dont l'autre extrémité est reliée à une prise de connexion 15.

Les rainures 6 sont au nombre de 9 et constituent des électrodes. Elles sont remplies d'un liquide physiologique 12 conducteur d'électricité dont la composition est appropriée au maintien en survie de la préparation neurobiologique 7 pendant une durée pouvant varier de cinq à dix heures. L'irrigation effectuée a pour fonction d'apporter de l'oxygène, des substrats énergétiques (glucose) et des ions à la préparation 7, en outre pour éviter sa dessication. La préparation 7 absorbe le liquide 12 par diffusion. Il est également prévu de pouvoir placer un couvercle (non représenté) sur l'ensemble de la préparation 7 afin d'assurer une protection prolongée contre la dessication.

Chaque extrémité d'électrode 6 est constituée par de petits "puits" faisant office de réservoirs 13 de liquide physiologique 12.

Selon un mode de mise en oeuvre préférentiel de l'invention, la préparation neurobiologique 7 est constituée par un ensemble "cerques 8 - nerfs cercaux 16 - sixième ganglion abdominal 17 - chaîne nerveuse 18" d'une blatte adulte. Les cerques 8 ainsi que l'extrémité 9 de la chaîne nerveuse 18 de la préparation neurobiologique 7 reposent dans des cuvettes 10,11 gravées dans la plaque horizontale 2. Chaque cuvette 10,11 contient du liquide physiologique 12 empêchant la dessication de la préparation 7. Le sixième ganglion abdominal 17 est situé entre les nerfs cercaux 16 et la chaîne nerveuse 18 et constitue une synapse entre les nerfs cercaux sensoriels et la chaîne nerveuse. Cette synapse 17 est logée dans une zone de réception gravée dans la rainure de l'électrode médiane 19 par le liquide physiologique 12. L'alimentation en liquide de cette électrode est réalisée par un cathéter 20 en polyvinyl relié à un réservoir 21 monté sur une potence 22 annexe. Cette irrigation continue est indispensable pour maintenir la transmission synaptique. Le débit de perfusion est réglable par déplacement vertical du réservoir 21 sur la potence 22. En mode de fonctionnement normal, le débit est d'environ 0,2 ml/min. Le liquide physiologique 12 circulant dans la rainure 19 est récupéré sous la cuve et s'échappe par un conduit 26 raccordé à l'électrode mixte 19.

Chaque paire de réservoirs 13 de liquide physiologique 12 comprend une extrémité d'un fil 14 conducteur d'électricité, avantageusement constitué en argent. Un fil d'argent 14 présente l'avantage d'être souple et de peu s'oxyder, notamment par rapport à un conducteur en cuivre. De plus, un tel matériau conducteur ne présente pas de toxicité pour la préparation neurobiologique. La combinaison de la rainure 6 et du fil 14 conducteur constitue une électrode mixte.

L'autre extrémité de chaque fil est reliée à une prise femelle 15 de connexion. Les neuf prises de connexion 15 sont avantageusement solidaires de la plaque frontale 3. Quatre de ces bornes 15 permettent ainsi un contact électrique avec la chaîne nerveuse 18 de la préparation 7, quatre avec les nerfs cercaux 16 et la dernière avec le sixième ganglion abdominal 17.

Des moyens 23 de stimulation et de lecture sont connectables aux neuf prises de connexion 15. On peut par exemple y connecter un stimulateur physiologique, un oscilloscope et/ou des appareils de traitement des signaux bioélectriques dérivés en divers points de la préparation neurobiologique 7 tel qu'un microordinateur. A titre d'exemple, les moyens 23 peuvent déterminer, et comparer les amplitudes et les temps de propagation des signaux bioelectriques en fonction des points de stimulation et des points de lecture.

Le dispositif selon l'invention permet de combiner à volonté les couples d'électrodes 6 et donc d'envoyer des stimuli à la préparation 7 maintenue en état de survie et de prélever des signaux bioélectriques en différents points de la préparation.

Le nombre d'électrodes 6 est spécialement prévu pour l'étude de la synapse 17 du sixième ganglion abdominal de la blatte et les nerfs cercaux 16 sont des moyens permettant l'étude de cette synapse 17.

L'électrode 19 en contact avec la synapse 17 permet notamment d'étudier les propriétés neurochimiques de la synapse 17. Il faut noter que la présente invention permet également de relever des signaux électriques provoqués par des stimuli mécaniques des mécano-récepteurs dont sont pourvus les cerques 8. Ces messages sensoriels peuvent par exemple être provoqués par un stimulus naturel (souffle d'air, choc mécanique).

Ajoutons que le dispositif selon l'invention peut aussi servir à étudier les réactions de la préparation 7 à des substances chimiques, que les moyens mis en oeuvre sont d'une grande simplicité et permettent une accessibilité aisée à la préparation 7, cette dernière étant simplement posée sur les électrodes 6.

Selon un mode de réalisation préférentiel de la présente invention, la plaque horizontale 2 faisant office de plan de repos à la préparation 7 comporte également quatre rainures 25 sensiblement parallèles et transversales à la direction d'extension de la préparation 7. Ces rainures 25 sont également pourvues d'une réserve de liquide physiologique et sont situées de part et d'autre de la synapse 17.

Une cuve à synapse selon l'invention est avantageusement destinée à l'expérimentation des synapses et de la conductivité nerveuse chez la blatte dans l'enseignement de la physiologie animale ou dans le cadre de recherches.

Bien entendu, d'autres applications de la cuve telle que décrite apparaîtront aisément à l'homme du métier sans pour autant sortir du cadre de la présente revendication indépendante.

## Revendications

1. Cuve (1) à organe isolé pour préparation neurobiologique, du type comprenant une plaque (2) horizontale d'extension d'une préparation neurobiologique (7), notamment destinée à un usage d'enseignement ou de recherche, coopérant avec des moyens d'alimentation de la préparation (7) en liquide (12) physiologique, et des moyens (23) de stimulation électrique et/ou de lecture des signaux bioélectriques de la préparation (7),
caractérisée en ce que les moyens (6,12,13,14) de stimulation et/ou de lecture comprennent au moins une électrode mixte, constituée d'une rainure (6) creusée dans ladite plaque (2) de réception de ladite préparation (7), sensiblement transversale à la direction d'extension de ladite préparation (7), ladite rainure (6) communiquant avec un réservoir-tampon (13) de liquide physiologique (12) conducteur recevant un fil (14) conducteur électrique de connexion auxdits moyens (23) de stimulation et/ou de lecture.

2. Cuve suivant la revendication 1 caractérisée en ce que lesdits moyens d'alimentation de la préparation (7) en liquide physiologique (12) sont constitués par au moins une rainure (6) creusée dans ladite plaque (2) de réception de ladite préparation (7), sensiblement transversale à la direction d'extension de ladite préparation (7), communicant avec un réservoir-tampon (13) de stockage dudit liquide physiologique (12).

3. Cuve selon l'une quelconque des revendications 1 et 2 caractérisé en ce que lesdits moyens d'alimentation de la préparation en liquide physiologique (12) comprennent au moins une cuvette (10,11) creusée dans ladite plaque (2) de réception de ladite préparation (7), destinée chacune à recevoir une extrémité (8,9) de ladite préparation (7), et contenant une réserve de liquide physiologique (12).

4. Cuve selon l'une quelconque des revendications 1 à 3 caractérisée en ce qu'elle comporte une rainure (19) creusée dans ladite plaque (2) de réception de ladite préparation (7), sensiblement transversale à la direction d'extension de ladite préparation (7), et comportant une zone de réception de la synapse (17) de ladite préparation (7), ladite rainure (19) communiquant avec des moyens (20,21,22) d'alimentation continue de ladite zone de réception de la synapse (17) par un liquide (12) physiologique à effet neurochimique en écoulement continu.

5. Cuve selon l'une quelconque des revendications 1 à 4 caractérisée en ce qu'elle comporte une pluralité de rainures (6) creusées dans ladite plaque (2) de réception de ladite préparation (7), sensiblement parallèles entre elles et transversales à la direction d'extension de ladite préparation (7), lesdites rainures (6) comprenant d'une part une rainure centrale (19) comprenant une zone de réception de ladite synapse (17) de ladite préparation (7), et d'autre part au moins quatre rainures d'électrode de part et d'autre de ladite rainure centrale (19).

6. Cuve selon l'une quelconque des revendications 1 à 5 caractérisée en ce que lesdits fils (14) conducteurs électriques constitutifs desdites électrodes sont terminés chacun par une prise (15) de connexion d'appareils (23), disposées de façon contigue sur une plaque frontale (3) commune de connexion.

7. Cuve selon la revendication 6 caractérisée en ce que lesdites prises (15) de connexion sont connectables sélectivement par paires, auxdits moyens (23) de stimulation et/ou de lecture, selon un grand nombre de combinaisons d'appariement.

8. Cuve selon l'une quelconque des revendications 4 à 7 caractérisée en ce qu'elle comprend une potence (22) support de suspension, à hauteur réglable, d'un réservoir (21) de liquide (12) formant lesdits moyens d'alimentation en continu de ladite rainure (19), comprenant une zone de réception de la synapse (17) de ladite préparation (7).

9. Application de la cuve selon l'une quelconque des revendications 1 à 8 à la réception de préparation neurobiologique de la blatte, comprenant : "cerques (8), nerfs cercaux (16), synapse (17) du sixième ganglion abdominal, chaîne nerveuse (18)".

10. Ensemble constitué d'une cuve selon l'une quelconque des revendications 1 à 9 associé à des moyens (23) de stimulation et/ou de lecture et d'exploitation électrique.

## Claims

1. Isolated organ vessel (1) for neurobiological preparation, of the type comprising a horizontal plate (2) for extension of a neurobiological preparation (7), especially intended for teaching or research use, cooperating with means for supplying the preparation (7) with physiological liquid (12), and means (23) for electrical stimulation and/or reading of the bioelectrical signals of the preparation (7),
characterized in that the means (6, 12, 13, 14) for stimulation and/or reading comprise at least one mixed electrode, constituted by a groove (6) hollowed into the said plate (2) for receiving the said preparation (7), substantially transversely to the direction of extension of the said preparation (7), the said groove (6) communicating with a buffer reservoir (13) of conductive physiological liquid (12) receiving an electrical conductor wire (14) for connection to the said means (23) for stimulation and/or reading.

2. Vessel according to Claim 1, characterized in that the said means for supplying the preparation (7) with physiological liquid (12) are constituted by at least one groove (6) hollowed into the said plate (2) for receiving the said preparation (7), substantially transversely to the direction of extension of the said preparation (7), communicating with a buffer reservoir (13) for storing the said physiological liquid (12).

3. Vessel according to any one of Claims 1 and 2, characterized in that the said means for supplying the preparation with physiological liquid (12) comprise at least one tray (10, 11) hollowed into the said plate (2) for receiving the said preparation (7), each intended to receive an end (8, 9) of the said preparation (7), and containing a reserve of physiological liquid (12).

4. Vessel according to any one of Claims 1 to 3, characterized in that it has a groove (19) hollowed into the said plate (2) for receiving the said preparation (7), substantially transversely to the direction of extension of the said preparation (7), and including a zone for receiving the synapse (17) of the said preparation (7), the said groove (19) communicating with means (20, 21, 22) for continuously supplying the said zone for receiving the synapse (17) with a physiological liquid (12) with neurochemical effect in a continuous flow.

5. Vessel according to any one of Claims 1 to 4, characterized in that it has a plurality of grooves (6) hollowed into the said plate (2) for receiving the said preparation (7), substantially parallel to one another and transversely to the direction of extension of the said preparation (7), the said grooves (6) comprising on the one hand a central groove (19) comprising a zone for receiving the said synapse (17) of the said preparation (7), and on the other hand at least four electrode grooves on either side of the said central groove (19).

6. Vessel according to any one of Claims 1 to 5, characterized in that the said electrical conductor wires (14) constituting the said electrodes are each terminated by a socket (15) for connection of apparatuses (23), arranged contiguously on a common front connection plate (3).

7. Vessel according to Claim 6, characterised in that the said connection sockets (15) are selectively connectable in pairs to the said means (23) for stimulation and/or reading, according to a large number of pairing combinations.

8. Vessel according to any one of Claims 4 to 7, characterized in that it comprises a suspension support bracket (22), adjustable in height, for a reservoir (21) of liquid (12) forming the said means for continuously supplying the said groove (19), comprising a zone for receiving the synapse (17) of the said preparation (7).

9. Application of the vessel according to any one of Claims 1 to 8 to receiving a neurobiological preparation of the cockroach, comprising: "cercus (8), cercal nerves (16), synapse (17) of the sixth abdominal ganglion, nerve chain (18)"

10. Assembly constituted by a vessel according to any one of Claims 1 to 9 associated with means (23) for stimulation and / or reading and electrical operation.

## Patentansprüche

1. Gefäß (1) zur Aufnahme abgetrennter Organe von der Art, die eine horizontale Platte (2) für das Ausbreiten eines neurobiologischen Präparats (7) umfaßt, besonders zu Lehr- oder Forschungszwecken geeignet, das mit Mitteln zur Versorgung des Präparats (7) mit physiologischer Flüssigkeit (12) sowie mit Mitteln (23) zur elektrischen Stimulierung und/oder zum Lesen bioelektrischer Präparatsignale zusammenwirkt,
dadurch gekennzeichnet, daß die Mittel (6, 12 , 13, 14) zum Stimulieren und/oder zum Lesen mindestens eine gemischte Elektrode umfassen, bestehend aus einer Rille (6), die in die Platte (2) zur Aufnahme des Präparats (7) eingearbeitet ist und in etwa quer zur Ausbreitungsrichtung des Präparats (7) verläuft, wobei die Rille (6) mit einem Puffergefäß (13) für die leitfähige physiologische Flüssigkeit (12) kommuniziert, in die ein elektrisch leitfähiger Draht (14) taucht, der die Verbindung zu den Mitteln (23) zur Stimulierung und/oder zum Lesen herstellt.

2. Gefäß gemäß Anspruch 1,
dadurch gekennzeichnet daß die Mittel zur Versorgung des Präparats (7) mit physiologischer Flüssigkeit (12) aus mindestens einer Rille (6) bestehen, die in die Aufnahmeplatte (2) für das Präparat (2) eingearbeitet ist und in etwa quer zur Ausbreitungsrichtung des Präparats (7) verläuft, wobei sie mit einem Puffergefäß (13) für die Aufbewahrung der physiologischen Flüssigkeit (12) kommuniziert.

3. Gefäß gemäß einem der Ansprüche 1 und 2,
dadurch gekennzeichnet, daß die Mittel zur Versorgung mit physiologischer Flüssigkeit (12) mindestens eine Mulde (10, 11) umfassen, die in die Aufnahmeplatte (2) für das Präparat (7) eingearbeitet ist, wobei diese Mulde(n) jeweils ein Ende (8, 9) des Präparats aufnimmt (aufnehmen) und eine Reservevorrat physiologischer Flüssigkeit enthält (enthalten).

4. Gefäß gemäß einem der Ansprüche 1 bis 3,
dadurch gekennzeichnet, daß es über eine Rille (19) verfügt, die in die Aufnahmeplatte (2) für das Präparat (7) eingearbeitet ist, in etwa quer zur Ausbreitungsrichtung des Präparats (7) verläuft und einen Bereich für die Aufnahme der Synapse (17) des Präparats (7) aufweist, wobei die Rille (19) mit Mitteln (20, 21, 22) kommuniziert zur kontinuierlichen Versorgung des Aufnahmebereichs der Synapse (17) mit einer physiologischen Flüssigkeit (12), die bei kontinuierlichem Fließen einen neurochemischen Effekt ausübt.

5. Gefäß gemäß einem der Ansprüche 1 bis 4,
dadurch gekennzeichnet, daß es eine Vielfalt von Rillen (6) umfaßt, die in die Aufnahmeplatte (2) für das Präparat (7) eingearbeitet sind, in etwa parallel zueinander und quer zur Ausbreitungsrichtung des Präparats (7) verlaufen, wobei diese Rillen (6) eine zentrale Rille (19) umfassen, die über einen Aufnahmebereich für die Synapse (17) des Präparats (7) verfügt und darüber hinaus mindestens vier Elektrodenrillen, die auf beiden Seiten der zentralen Rille (19) verlaufen.

6. Gefäß gemäß einem der Ansprüche 1 bis 5,
dadurch gekennzeichnet, daß die elektrisch leitenden Drähte (14), die Bestandteil der Elektroden sind, jeweils mit einer Anschlußbuchse (15) für Geräte (23) enden, die nebeneinander auf einer gemeinsamen Anschlußfrontplatte (3) angebracht sind.

7. Gefäß gemäß Anspruch 6,
dadurch gekennzeichnet, daß die Anschlußbuchsen (15) paarweise selektiv an die Mittel (23) zur Stimulierung und/oder zum Lesen anschließbar sind, wobei eine große Anzahl von Paarbildungen möglich ist.

8. Gefäß gemäß einem der Ansprüche 4 bis 7,
dadurch gekennzeichnet, daß es über ein höhenverstellbares Trägergestell (22) für ein Gefäß (21) verfügt, welches eine Flüssigkeit (12) enthält, welches die Mittel zur kontinuierlichen Versorgung der Rille (19) bildet, die einen Bereich zum Empfang der Synapse (17) des Präparats (7) aufweist.

9. Anwendung des Gefäßes gemäß einem der Ansprüche 1 bis 8 auf die Aufnahme eines neurobiologischen Schabenpräparats,
welches folgendes umfaßt: ''Nerventeile (8), Ringnerven (16), Synapse (17) des sechsten Bauchnervenknotens, Nervenstrang (18)''.

10. Vorrichtung, bestehend aus einem Gefäß gemäß einem der Ansprüche 1 bis 9, das mit Mitteln (23) zur Stimulierung und/oder zum Lesen sowie zum elektrischen Betrieb verbunden ist.
